# EUROPEAN PATENT SPECIFICATION

(11) **EP 2 542 251 B1**
(45) Date of publication and mention of the grant of the patent: **01.11.2017**
(21) Application number: 11751210.3
(22) Date of filing: 01.03.2011
(51) Int. Cl.: A61K 36/48, A61P 35/00, A61K 36/185

(54) **SANDALWOOD OIL AND ITS USES**
SANDELHOLZÖL UND DESSEN VERWENDUNGEN
HUILE DE BOIS DE SANTAL ET SES UTILISATIONS

(30) Priority: 01.03.2010 US 309183 P
(43) Date of publication of application: 09.01.2013
(73) Proprietor: Santalis Pharmaceuticals Inc., San Antonio, TX 78249 (US)
(72) Inventor: CLEMENTS, Ian, San Antonio Texas 78249 (US); CASTELLA, Paul, San Antonio Texas 78249 (US); LEVENSON, Corey, San Antonio Texas 78249 (US)
(74) Representative: Leißler-Gerstl, Gabriele
(86) International application number: PCT/US2011/026706
(87) International publication number: WO 2011/109411

(56) References cited:
- EP-A1- 1 402 785
- US-A- 6 132 756
- US-A1- 2009 047 372
- BURDOCK ET AL: "Safety assessment of sandalwood oil (Santalum album L.)", FOOD AND CHEMICAL TOXICOLOGY, vol. 46, no. 2, 21 December 2007 (2007-12-21), pages 421-432, XP022399085, PERGAMON, GB ISSN: 0278-6915, DOI: 10.1016/J.FCT.2007.09.092
- KAUR MANJINDER ET AL: "Skin cancer chemopreventive agent, alpha-santalol, induces apoptotic death of human epidermoid carcinoma A431 cells via caspase activation together with dissipation of mitochondrial membrane potential and cytochrome c release", CARCINOGENESIS, vol. 26, no. 2, 1 February 2005 (2005-02-01), pages 369-380, XP002593567, OXFORD UNIVERSITY PRESS, GB ISSN: 0143-3334
- SINDHU R K ET AL: "Santalum album linn: A review on morphology, phytochemistry and pharmacological aspects", INTERNATIONAL JOURNAL OF PHARMTECH RESEARCH, vol. 2, no. 1, January 2010 (2010-01), pages 914-919, XP009171560, SPHINX KNOWLEDGE HOUSE IND ISSN: 0974-4304
- DATABASE TCM SIPO; 15 September 1992 (1992-09-15), Pan Haibin: "A medicated core of bra having anticancer and breast beautifying effects and its preparation method", XP002707360, Database accession no. CN-92110783-A & CN 1 084 065 A (PAN HAIBIN [CN]) 23 March 1994 (1994-03-23)
- DATABASE WPI Week 200636 Thomson Scientific, London, GB; AN 2006-347100 XP002707361, & JP 2006 124296 A (ALPS YAKUHIN KOGYO KK) 18 May 2006 (2006-05-18)
- ERLIGMANN A: "Sandalwood Oils", INTERNATIONAL JOURNAL OF AROMATHERAPY, vol. 11, no. 4, 2001, pages 186-192, XP009171559, GB ISSN: 0962-4562
- DWIVEDI C ET AL: "CHEMOPREVENTIVE EFFECTS OF SANDALWOOD OIL ON SKIN PAPILLOMAS IN MICE", EUROPEAN JOURNAL OF CANCER PREVENTION, vol. 6, no. 4, 1 August 1997 (1997-08-01), pages 399-401, XP001121309, LIPPINCOTT WILLIAMS & WILKINS, PHILADELPHIA, US ISSN: 0959-8278

## Description

### BACKGROUND

Cancer is characterized by the rapid creation of abnormal cells that grow beyond normal cellular boundaries and beyond normal cellular rates of growth. Cancer is a leading cause of death worldwide and accounted for 7.6 million deaths in 2008.

### SUMMARY

**P**rovided herein are therapeutically effective compositions as defined in the claims of sandalwood oil and kits comprising the compositions. Also provided are methods of making and using the compositions. More specifically, disclosed herein is a method of treating a non-skin cancer in a subject, said method comprising administering to the subject an effective amount of a composition comprising sandalwood oil, wherein the subject has a non-skin cancer.

### BRIEF DESCRIPTION OF DRAWINGS

Figure 1 (for reference) shows the effect of Australian sandalwood oil on a C8161 human melanoma cell line.
Figure 2 (for reference) shows the effect of East Indian sandalwood oil on a C8161 human melanoma cell line.
Figure 3 shows the effect of Australian sandalwood oil on a FA**DU** human head and neck cancer cell line.
Figure 4 shows the effect of East Indian sandalwood oil on a FADU human head and neck cancer cell line.
Figure 5 shows the effect of Australian sandalwood oil on a HELA human cervical cancer cell line.
Figure 6 shows the effect of East Indian sandalwood oil on a HELA human cervical cancer cell line.
Figure 7 shows the effect of Australian sandalwood oil on a MIA PACA-2 human pancreatic cancer cell line.
Figure 8 shows the effect of East Indian sandalwood oil on a MIA PACA-2 human pancreatic cancer cell line.
Figure 9 shows the effect of Australian sandalwood oil on a SNU-398 human hepatocellular carcinoma (liver) cell line.
Figure 10 shows the effect of East Indian sandalwood oil on a SNU-398 human hepatocellular carcinoma (liver) cell line.
Figure 11 shows the effects of Australian sandalwood oil and East Indian sandalwood oil on toxicity in human MRC5 (normal human fetal lung fibroblast) cells after 20 hours.

### DETAILED DESCRIPTION OF THE INVENTION

Sandalwood is the name of various fragrant woods from the genus *Santalum,* which contain essential oil. The wood is heavy and yellow in color as well as fine-grained, and unlike many other aromatic woods it retains its fragrance for decades. The genuine sandalwoods are medium-sized hemiparasitic trees. In the present methods, oil from any member of the genus *Santalum* can be used. For example, and not to be limiting, East Indian sandalwood (*Santalum album*) or West Australian sandalwood (*Santalum spicatum*) can be utilized in the methods set forth herein. Several other members of the genus species also have fragrant wood and are found across India, Australia, Indonesia, and the Pacific Islands.

*Santalum album,* or East Indian sandalwood, is currently a vulnerable species in the wild and consequently very expensive. Although all sandalwood trees in India and Nepal are government-owned and their harvest from the wild is strictly controlled. Commercial *Santalum album* plantations have been established in Western Australia over the last 15 years that have allowed establishment of a sustainable and consistent supply of oil. *Santalum ellipticum, S. freycinetianum,* and *S. paniculatum,* the Hawaiian sandalwoods, can also be used.

As set forth above, *Santalum spicatum* (West Australian sandalwood) can be used. The concentration of constituent chemicals in its essential oil differs from those of other *Santalum* species, for example, *S. album.* Other species produced in Australia that can be utilized in the methods , compositions, and kits set forth herein include, but are not limited to *S. acuminatum, S. lanceolatum, S. murrayanum, S. obtusifolium* and *S. album.*

The *S. spicatum* and *S. album* species have different fragrances, reflected by differences in their components. A comparison of the components of steam distilled Australian and Indian sandalwood oils is presented in Table 1. The components and their percentages can vary with the extraction method.

| **Table 1: Typical Sandalwood Oil Profiles** | | |
|---|---|---|
| **Compound** | ***S. spicatum* %** | ***S. album* %** |
| E nerolidol | 2.1% | 0.1% |
| Alpha-santalene | nd | 0.5% |
| Cis-alpha-(trans) bergamotene | nd | 0.7% |
| Epi-beta-santalene | nd | 1.1% |
| Beta-santalene | nd | 0.3% |
| Gamma-curcumene | nd | 0.2% |
| Dendrolasin | 1.2% | 0.2% |
| Alpha-santalol | 17.2% | 48.7% |
| Beta-bisabolol | 2.3% | 0.5% |
| Epi -alpha-bisabolol | 8% | nd |
| Z-alpha trans -bergamotol | 4.2% | 2.4% |
| Epi beta -santalol | 1.2% | 5% |
| Cis -beta-santalol | 11.4% | 20.4% |
| E,E, farnesol | 6.5% | nd |
| Cis nuciferol | 13.5% | 0.6% |
| Z-beta-curcumen-12-ol | 7.9% | 0.2% |
| cis lanceol | 2.9% | 1.5% |

There are several methods of producing oils from sandalwood. Generally, steam distillation processes are used, but solvent extraction and combinations thereof can also be used. Hydrodistillation is a traditional method of extraction. This method yields an aromatic oil. Instead of having steam pass through the powdered wood, in a hydrodistiller the powder is allowed to soak in water. A fire from below the vessel heats the water and carries off the steam, which is allowed to cool. The sandalwood oil is then removed from the top of the hydrosol.

Sandalwood essential oil can also be extracted by steam distillation, a process in which super-heated steam is passed through the powdered wood. The steam helps to release and carry away the essential oil that is locked in the cellular structure of the wood. The steam is then cooled and the result is sandalwood hydrosol and sandalwood essential oil.

Supercritical CO₂ extraction is another technique for extracting essential oils (and other constituents) from plant materials. It does not use water or steam, but instead supercritical CO₂ (carbon dioxide) is used as a solvent. This method allows the aromatic constituents to be extracted without heat, after which the CO₂ is removed from the resulting extract by evaporation and the oil is then refined and filtered. See M. J. Piggott, et al., Western Australian Sandalwood Oil: Extraction by Different Techniques and Variations of the Major Components in Different Sections of a Single Tree, Flavour and Fragrance Journal 12(1): 43 - 46 (1998). Producing commercially valuable sandalwood with high levels of fragrance oils, requires the trees to be at least eight years of age as a minimum, but fourteen years and older is preferable. Australia is now the largest producer of *Santalum spicatum* and Australia will be producing Indian sandalwood in commercial quantities in the next few years.

Unlike most trees, sandalwood is harvested by toppling the entire tree instead of sawing them down at the trunk. In this way, valuable wood from the stump and root can also be sold or processed for oil. The fact that the entire tree is harvested and used for oil production makes the oils produced remarkably consistent over the season and from one season to another. This is an important advantage when the tree is to be used for western pharmaceutical production, and compares favorably to pharmaceuticals derived from seasonal plants, or the seasonal part of other trees and bushes, such as their fruit, nuts, or leaves. Further improving the reproducibility of the oils is that fact that some of the extraction techniques have been standardized. *See e.g.,* ISO 3518:2002 and ISO 22769:2009. Furthermore, although oils extracted from commercial plantations can be utilized, the oils can also be extracted from cell culture or fermentation of tree cells.

As used herein, a sandalwood oil can be a sandalwood oil that conforms with International Organization for Standardization (ISO) specifications for the oil and therefore comprises 20-45% santalols, when derived from *S. spicatum,* and 57-79% santalols when derived from *S. album.* However, the 20-45% santalols and the 57-79% santalols are determined against the pure oil and before such oil is combined with any other excipients or active ingredients. It is understood that an efficacious preparation of sandalwood oil may have a concentration of santalols lower (or higher) than the sandalwood oil it is prepared from, and that the efficacious concentrations may be derived from sandalwood oils that are outside of the ISO specification prior to formulation. A santalol can be an α-santalol (shown below), a β-santalol (shown below), or and any other active isomers or derivatives (such as esters) thereof.

As used herein, a sandalwood oil can comprise at least about 20%, 25%, 30%, 35%, 40%, 45%, 50%, 55%, 60%, 65%, 70%, 75%, 80%, 85%, 90%, 95% , 99% santalols or any percentage in between the percentages set forth herein, when derived from *S. spicatum.* The sandalwood oil can comprise at least 45%, 50%, 55%, 60%, 65%, 70%, 75%, 80%, 85%, 90%, 95%, 99% santalols or any percentage in between the percentages set forth herein, when derived from *S. album.* As set forth above, the oil can be extracted from cultivated trees or from cell culture of tree cells.

In the methods set forth herein, the sandalwood oil can comprise the ingredients in the amounts listed in Table 1 plus or minus about 20%, and more preferably plus or minus about 10%, 5%, 2% ,1% or any percentage in between the percentages set forth herein. The composition can comprise pharmaceutically acceptable excipients or diluents. The composition can also comprise other active ingredients in addition to sandalwood oil.

It is also understood that the activity of sandalwood oil can be due to one or more components set forth in Table 1 acting either separately or together. Therefore, formulations that increase the concentration of the active component(s) and reduce the concentration of the inactive component(s) are set forth herein. Synthetic versions of the active components, or their derivatives, may be formulated in conjunction with or to replace the naturally occurring components of sandalwood oil.

The sandalwood oil can be prepared by steam distillation, supercritical CO₂ extraction, solvent extraction, hydro-distillation and combinations thereof. It is also possible to synthesize one or more of the active ingredients of sandalwood oil, as identified in Table 1 and thereafter combine individual active ingredients together.

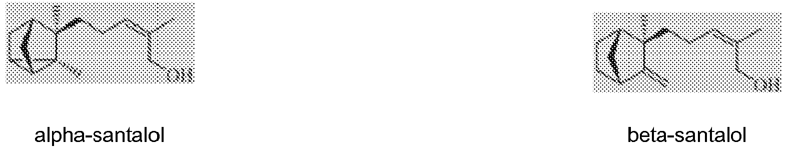

As used herein, the term subject can be a vertebrate, more specifically a mammal (e.g., a human, horse, pig, rabbit, dog, sheep, goat, non-human primate, cow, cat, guinea pig or rodent), a fish, a bird or a reptile or an amphibian. The term does not denote a particular age or sex. Thus, adult and newborn subjects, whether male or female, are intended to be covered. As used herein, patient or subject may be used interchangeably and can refer to a subject with a disease or disorder. The term patient or subject includes human and veterinary subjects.

The disclosed method of treating cancer in a subject comprises administering to the subject an effective amount of the composition comprising sandalwood oil as defined in the claims, wherein the subject has a non-skin cancer. Any non-skin cancer can be treated by the methods set forth herein. These include, but are not limited to, pancreatic cancer, breast cancer, brain cancer (e.g., glioblastoma), lung cancer, prostate cancer, bladder cancer, a central nervous system cancer, ovarian cancer, head and neck cancer, colorectal cancer, thyroid cancer, renal cancer, adrenal cancer, liver cancer, and leukemia. The cancer can be a solid neoplasm (e.g., sarcoma or carcinoma) or a cancerous growth affecting the hematopoietic system (e.g., lymphoma or leukemia). The present method of treating a non-skin cancer in a subject comprises administering to the subject an effective amount of sandalwood oil, wherein the cancer in the subject is not a cervical cancer.

As used herein the terms treatment, treat, treating or ameliorating refers to a method of reducing the effects of a disease or condition or symptom of the disease or condition. Thus in the disclosed method, treatment can refer to a 10%, 20%, 30%, 40%, 50%, 60%, 70%, 80%, 90%, or 100% reduction or amelioration in the severity of an established disease or condition or symptom of the disease or condition. For example, the method for treating cancer is considered to be a treatment if there is a 10% reduction in one or more symptoms of the disease in a subject as compared to a control subject that did not receive a composition comprising sandalwood oil. Thus the reduction can be a 10, 20, 30, 40, 50, 60, 70, 80, 90, 100%, or any percent reduction in between 10 and 100 as compared to control levels. It is understood that treatment does not necessarily refer to a cure or complete ablation of the disease, condition, or symptoms of the disease or condition.

Further disclosed for reference is a method of preventing the progression of actinic keratosis to squamous cell carcinoma (SCC) in a subject, comprising administering to a subject an effective amount of sandalwood oil. As utilized herein, by "prevent," "preventing," or "prevention" is meant a method of precluding, delaying, averting, obviating, forestalling, stopping, or hindering the onset or incidence of the progression of actinic keratosis to squamous skin cell carcinoma. For example, the disclosed method is considered to be a prevention if there is about a 10% reduction or delay in onset of SCC or progression of actinic keratosis to SCC in a subject when compared to control subjects with actinic keratosis that did not receive a composition for preventing SCC. Thus, the reduction can be about a 10, 20, 30, 40, 50, 60, 70, 80, 90, 100%, or any amount of reduction in between as compared to control subjects.

The mode of action of the ingredients contained in sandalwood oil can be apoptotic. Therefore, it can be advantageous to combine sandalwood oil, or the ingredients therein, with a chemotherapeutic agent. These chemotherapeutic agents include, but are not limited to, Acivicin; Aclarubicin; Acodazole Hydrochloride; AcrQnine; Adozelesin; Aldesleukin; Altretamine; Ambomycin; Ametantrone Acetate; Aminoglutethimide; Amsacrine; Anastrozole; Anthramycin; Asparaginase; Asperlin; Azacitidine; Azetepa; Azotomycin; Batimastat; Benzodepa; Bicalutamide; Bisantrene Hydrochloride; Bisnafide Dimesylate; Bizelesin; Bleomycin Sulfate; Brequinar Sodium; Bropirimine; Busulfan; Cactinomycin; Calusterone; Caracemide; Carbetimer; Carboplatin; Carmustine; Carubicin Hydrochloride; Carzelesin; Cedefingol; Chlorambucil; Cirolemycin; Cisplatin; Cladribine; Crisnatol Mesylate; Cyclophosphamide; Cytarabine; Dacarbazine; Dactinomycin; Daunorubicin Hydrochloride; Decitabine; Dexormaplatin; Dezaguanine; Dezaguanine Mesylate; Diaziquone; Docetaxel; Doxorubicin; Doxorubicin Hydrochloride; Droloxifene; Droloxifene Citrate; Dromostanolone Propionate; Duazomycin; Edatrexate; Eflomithine Hydrochloride; Elsamitrucin; Enloplatin; Enpromate; Epipropidine; Epirubicin; Epirubicin Hydrochloride; Erbulozole; Esorubicin Hydrochloride; Estramustine; Estramustine Phosphate Sodium; Etanidazole; Ethiodized Oil I 131; Etoposide; Etoposide Phosphate; Etoprine; Fadrozole Hydrochloride; Fazarabine; Fenretinide; Floxuridine; Fludarabine Phosphate; 5-Fluorouracil; Flurocitabine; Fosquidone; Fostriecin Sodium; Gemcitabine; Gemcitabine Hydrochloride; Gold Au 198; Hydroxyurea; Idarubicin Hydrochloride; Ifosfamide; Ilmofosine; Interferon Alfa-2a; Interferon Alfa-2b; Interferon Alfa-n1; Interferon Alfa-n3; Interferon Beta- I a; Interferon Gamma- Ib; Iproplatin; Irinotecan Hydrochloride; Lanreotide Acetate; Letrozole; Leuprolide Acetate; Liarozole Hydrochloride; Lometrexol Sodium; Lomustine; Losoxantrone Hydrochloride; Masoprocol; Maytansine; Mechlorethamine Hydrochloride; Megestrol Acetate; Melengestrol Acetate; Melphalan; Menogaril; Mercaptopurine; Methotrexate; Methotrexate Sodium; Metoprine; Meturedepa; Mitindomide; Mitocarcin; Mitocromin; Mitogillin; Mitomalcin; Mitomycin C; Mitosper; Mitotane; Mitoxantrone; Mitoxantrone Hydrochloride; Mycophenolic Acid; Nocodazole; Nogalamycin; Ormaplatin; Oxisuran; Paclitaxel; Pegaspargase; Peliomycin; Pentamustine; Peplomycin Sulfate; Perfosfamide; Pipobroman; Piposulfan; Piroxantrone Hydrochloride; Plicamycin; Plomestane; Porfimer Sodium; Porfiromycin; Prednimustine; Procarbazine Hydrochloride; Puromycin; Puromycin Hydrochloride; Pyrazofurin; Riboprine; Rogletimide; Safmgol; Safingol Hydrochloride; Semustine; Simtrazene; Sparfosate Sodium; Sparsomycin; Spirogermanium Hydrochloride; Spiromustine; Spiroplatin; Streptonigrin; Streptozocin; Strontium Chloride Sr 89; Sulofenur; Talisomycin; Taxane; Taxoid; Tecogalan Sodium; Tegafur; Teloxantrone Hydrochloride; Temoporfin; Teniposide; Teroxirone; Testolactone; Thiamiprine; Thioguanine; Thiotepa; Tiazofurin; Tirapazamine; Topotecan Hydrochloride; Toremifene Citrate; Trestolone Acetate; Triciribine Phosphate; Trimetrexate; Trimetrexate Glucuronate; Triptorelin; Tubulozole Hydrochloride; Uracil Mustard; Uredepa; Vapreotide; Verteporfin; Vinblastine Sulfate; Vincristine Sulfate; Vindesine; Vindesine Sulfate; Vinepidine Sulfate; Vinglycinate Sulfate; Vinleurosine Sulfate; Vinorelbine Tartrate; Vinrosidine Sulfate; Vinzolidine Sulfate; Vorozole; Zeniplatin; Zinostatin; Zorubicin Hydrochloride. A combination of a sandalwood oil composition with a chemotherapeutic agent reduces the dosage generally required for either agent along. This is highly desirable, as such reduction would concomitantly reduce toxicity caused by higher doses of either the sandalwood oil composition or the chemotherapeutic agent.

The sandalwood oils or ingredients thereof can be provided in a pharmaceutical composition. Depending on the intended mode of administration, the pharmaceutical composition can be in the form of solid, semi-solid or liquid dosage forms, such as, for example, tablets, suppositories, pills, capsules, powders, liquids, or suspensions, preferably in unit dosage form suitable for single administration of a precise dosage. The compositions will include a therapeutically effective amount of the sandalwood oil in combination with a pharmaceutically acceptable carrier and, in addition, may include other medicinal agents, pharmaceutical agents, carriers, or diluents. By pharmaceutically acceptable carrier is meant a material that is not biologically or otherwise undesirable, which can be administered to an individual along with the selected compound without causing unacceptable biological effects or interacting in a deleterious manner with the other components of the pharmaceutical composition in which it is contained.

Various delivery systems for administering the compositions disclosed herein are known, and include encapsulation in liposomes, microparticles or microcapsules. Methods of introduction include, but are not limited to, mucosal, topical, intradermal, intrathecal, intratracheal, via nebulizer, via inhalation, intravesicular, intramuscular, intraperitoneal, vaginal, rectal, intravenous, subcutaneous, intranasal, and oral routes. Combinations of administration can also be utilized. For example, a composition can be delivered intranasally and intravenously to the subject. In another example, a composition can be administered orally and intravenously to the subject. Compounds can be administered by any convenient route, for example by infusion or bolus injection, by absorption through epithelial or mucocutaneous linings (for example, oral mucosa, rectal, vaginal and intestinal mucosa, etc.) and can be administered together with other biologically active agents. Administration can be systemic or local. Pharmaceutical compositions can be delivered locally to the area in need of treatment, for example by topical application or local injection, such as injection directly into a tumor. The mode of delivery is determined empirically based on a number of factors including the type of cancer.

For all of the administration methods disclosed herein, each method can optionally further comprise the step of diagnosing a subject with cancer or diagnosing a subject in need of prophylaxis or prevention of squamous cell carcinoma. The method can also include assessing the effectiveness of the sandalwood oil composition, optionally in combination with the chemotherapeutic agent, and modifying the treatment regimen.

The amount of therapeutic agent effective in treating cancer can depend on the nature of the cancer and its associated symptoms, and can be determined by standard clinical techniques. Therefore, these amounts will vary depending on the type of cancer. In addition, *in nitro* assays can be employed to identify optimal dosage ranges. The precise dose to be employed in the formulation will also depend on the route of administration, and the seriousness of the disease or disorder, and should be decided according to the judgment of the practitioner and each subject's circumstances. For example, the concentration of sandalwood oil in the composition administered to the subject can be from about 0.5 micromolar to about 300 micromolar. In other examples, the concentration of sandalwood oil can be from about 1 micromolar to about 150 micromolar, from about 5 micromolar to about 150 micromolar, from about 10 micromolar to about 150 micromolar, from about 20 micromolar to about 150 micromolar, from about 30 micromolar to about 150 micromolar, from about 40 micromolar to about 150 micromolar, from about 50 micromolar to about 150 micromolar, 60 micromolar to about 150 micromolar, 70 micromolar to about 150 micromolar, 80 micromolar to about 150 micromolar, 90 micromolar to about 150 micromolar, 100 micromolar to 150 micromolar, 125 micromolar to 150 micromolar, 1 micromolar to about 300 micromolar, from about 5 micromolar to about 300 micromolar, from about 10 micromolar to about 300 micromolar, from about 20 micromolar to about 300 micromolar, from about 30 micromolar to about 300 micromolar, from about 40 micromolar to about 300 micromolar, from about 50 micromolar to about 300 micromolar, 60 micromolar to about 300 micromolar, 70 micromolar to about 300 micromolar, 80 micromolar to about 300 micromolar, 90 micromolar to about 300 micromolar, 100 micromolar to 300 micromolar, 125 micromolar to 300 micromolar, 150 micromolar to 300 micromolar, 175 micromolar to 300 micromolar, 200 micromolar to 300 micromolar, 225 micromolar to 300 micromolar, 250 micromolar to 300 micromolar or 275 micromolar to 300 micromolar. When concentrations of sandalwood oil are expressed in micromolarity, it is understood that this is the micromolarity of alpha-santalol in the sandalwood oil. For example, the concentration of sandalwood oil can be about 1, 5, 10, 15, 20, 25, 30, 35, 40, 45, 50, 55, 60, 65, 60, 75, 80, 85, 90, 95, 100, 105, 110, 115, 120, 125, 130, 135, 140, 145, 150,155,160, 165, 170, 175, 180, 185, 190, 195, 200, 205, 210, 215, 220, 225, 230, 235, 240, 245, 250, 255, 260, 265, 270, 275, 280, 285, 290, 295, 300 micromolar or any concentration in between the concentrations set forth herein. The concentration of sandalwood in the composition is 0002% to 0.1% sandalwood oil (w/w). For example, the concentration of sandalwood in the composition can be about .0002% to about .005%, or from about .0002% to about .01%, or from about .0002% to about .05%, or from about .0002% to about 0.1 %. The concentration of sandalwood oil can also be about .0002%, .0003%, .0004%, .0005%, .0006%, .0007%, .0008%, .0009%, 001%, .002%, .003%, .004%, .005%, .006%, .007%, .008%, .009%, .01%, .02%, .03%, .04%, .05%, .06%, .07%, .08%, .09%, 0.1% or any percentage in between the percentages set forth herein. Multiple administrations and/or dosages can also be used. Effective doses can be extrapolated from dose-response curves derived from *in vitro* or animal model test systems.

For any of the delivery mechanisms set forth herein, one of skill in the art can formulate the composition such that the concentration of sandalwood oil at the target organ is between about 0.5 micromolar to about 300 micromolar. This range is not meant to be limiting as the concentration at the target organ can be higher or lower depending on the delivery mechanism and the target organ. Depending on the formulation and the route of delivery, in order to achieve this concentration at the target organ, the dosage can range from about 0.01 mg/kg to about 100mg/kg. For example, the dosage can range from about 0.01 mg/kg to about 1mg/kg, from about 0.01mg/kg to about 5 mg/kg, from about 1mg/kg to about 5 mg/kg, from about 1 mg/kg to about 10 mg/kg, from about 1 mg/kg to about 25 mg/kg, from about 1 mg/kg to about 50 mg/kg, from about 1 mg/kg to 100 mg/kg or any other dosage in between the dosage amounts set forth herein.

The disclosure also provides a pharmaceutical pack or kit comprising one or more containers filled with one or more of the ingredients of the pharmaceutical compositions. Optionally associated with such container(s) in the kit is a mode of administration, including, for example, a syringe, an inhaler, or the like.

As used herein, the term carrier encompasses any excipient, diluent, filler, salt, buffer, stabilizer, solubilizer, lipid, stabilizer, or other material well known in the art for use in pharmaceutical formulations. The choice of a carrier for use in a composition will depend upon the intended route of administration for the composition. The preparation of pharmaceutically acceptable carriers and formulations containing these materials is described in, *e.g*., Remington's Pharmaceutical Sciences, 21st Edition, ed. University of the Sciences in Philadelphia, Lippincott, Williams & Wilkins, Philadelphia Pa., 2005. Examples of physiologically acceptable carriers include buffers such as phosphate buffers, citrate buffer, and buffers with other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN® (ICI, Inc.; Bridgewater, New Jersey), polyethylene glycol (PEG), and PLURONICS™ (BASF; Florham Park, NJ).

Compositions containing sandalwood oil suitable for parenteral injection may comprise physiologically acceptable sterile aqueous or nonaqueous solutions, dispersions, suspensions or emulsions, and sterile powders for reconstitution into sterile injectable solutions or dispersions. Compositions for parenteral injection can also include liposomes, emulsions or co-solvents. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

These compositions may also contain adjuvants such as preserving, wetting, emulsifying, and dispensing agents. Prevention of the action of microorganisms can be promoted by various antibacterial and antifungal agents, for example, parabens, chlorobutanol, phenol, sorbic acid, and the like. Isotonic agents, for example, sugars, sodium chloride, and the like may also be included. Prolonged absorption of the injectable pharmaceutical form can be brought about by the use of agents delaying absorption, for example, aluminum monostearate and gelatin.

Solid dosage forms for oral administration of the compounds described herein or derivatives thereof include capsules, tablets, pills, powders, and granules. In such solid dosage forms, the compounds described herein or derivatives thereof is admixed with at least one inert customary excipient (or carrier) such as sodium citrate or dicalcium phosphate or (a) fillers or extenders, as for example, starches, lactose, sucrose, glucose, mannitol, and silicic acid, (b) binders, as for example, carboxymethylcellulose, alignates, gelatin, polyvinylpyrrolidone, sucrose, and acacia, (c) humectants, as for example, glycerol, (d) disintegrating agents, as for example, agar-agar, calcium carbonate, potato or tapioca starch, alginic acid, certain complex silicates, and sodium carbonate, (e) solution retarders, as for example, paraffin, (f) absorption accelerators, as for example, quaternary ammonium compounds, (g) wetting agents, as for example, cetyl alcohol, and glycerol monostearate, (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents. Solid dosage forms such as hard or soft capsules can be utilized in the treatment of bladder cancer or any other cancer for which oral administration is desirable. Dosage ranges for these capsules are set forth above.

Solid compositions of a similar type may also be employed as fillers in soft and hard-filled gelatin capsules using such excipients as lactose or milk sugar as well as high molecular weight polyethyleneglycols, and the like.

Solid dosage forms such as tablets, dragees, capsules, pills, and granules can be prepared with coatings and shells, such as enteric coatings and others known in the art. They may contain opacifying agents and can also be of such composition that they release the active compound or compounds in a certain part of the intestinal tract in a delayed manner. Examples of embedding compositions that can be used are polymeric substances and waxes. The active compounds can also be in microencapsulated form, if appropriate, with one or more of the above-mentioned excipients.

Liquid dosage forms for oral administration of the compounds described herein or derivatives thereof include pharmaceutically acceptable emulsions, solutions, suspensions, syrups, and elixirs. In addition to the active compounds, the liquid dosage forms may contain inert diluents commonly used in the art, such as water or other solvents, solubilizing agents, and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil, sesame oil, glycerol, tetrahydrofurfuryl alcohol, polyethyleneglycols, and fatty acid esters of sorbitan, or mixtures of these substances, and the like.

Liposomes and nanoparticles can also be utilized for site-specific delivery of the compositions to an organ, for example, for delivery to the liver. Compositions for the treatment of nasal or lung cancers can be formulated in an aerosol or other inhalable form.

Ranges may be expressed herein as from about one particular value, and/or to about another particular value. When such a range is expressed, this includes from the one particular value and/or to the other particular value. Similarly, when values are expressed as approximations, by use of the antecedent about, it will be understood that the particular value is disclosed.

A number of aspects have been described. Nevertheless, it will be understood that various modifications may be made. Furthermore, when one characteristic or step is described it can be combined with any other characteristic or step herein even if the combination is not explicitly stated. Accordingly, other aspects are within the scope of the claims.

### EXAMPLES

For the experiments described herein two sandalwood oil extracts were studied. The oil was prepared by steam distillation and contained the components shown in Table 1.

### BREAST CANCER

For analysis of activity in breast cancer, several cell lines were assayed against the tumoricidal activity of the two sandalwood oils described above. Screening was performed by the Southern Research Institute (**B**irmingham AL) using protocols based on those developed by the Developmental Therapeutics Program of N**C**I/NIH. Briefly, the human tumor cell lines of the cancer screening panel are grown in RPMI 1640 medium (SIGMA ALDRICH®) containing 10% fetal bovine serum and 2 mM L-glutamine.

Once enough cells propagated, seven plates of all adherent lines were seeded at 5,000 cells/well in a total volume of 50 µL, and seven plates of all suspension lines were seeded at 5,000 - 20,000 cells/well (depending on cell line) in a total volume of 50 µL. Plates were placed in a humidified cell culture incubator overnight to allow adherent cells to attach.

50 µL of oil solutions were added at various concentrations to appropriate wells already containing 50 µL of cells and medium to expose cells to the final concentrations of compounds required. 50 µL of media was added to media and cell control wells and 50 µL of appropriate mix to the appropriate plates to vehicle control wells. At the same time as drug exposure, a CellTiter-Glo® Assay (CTG) assay was carried out on the Day 0 plate to obtain a Day 0 count. Cells are exposed to compounds for 72 hr. Following a 72 hr exposure period, all remaining plates were assayed using CTG.

The extent of growth inhibitory activity of each oil was reported as IC₅₀, which is the nominal concentration of oil that resulted in a cell growth at 72 hours that was 50% of that observed in the control wells, which contained cells but no oil samples. The results are presented below:

| Table 2. Activity against breast cancer cell lines | | | |
|---|---|---|---|
| Cell Line | Panel Name | IC₅₀ (µM) | |
| | | *Santalum album* | *Santalum spicatum* |
| MCF7 | Breast | 55 | 102 |
| MDA-MB-231/ATCC | Breast | 61 | 140 |
| MDA-MB-468 | Breast | 20 | 70 |
| HS 578T | Breast | 29 | 93 |
| BT-549 | Breast | 73 | 149 |
| T-47D | Breast | 38 | 102 |

Robust activity against 6 cell lines derived from human breast cancers was shown. This indicates broad activity against many human breast cancers. In particular, the growth of all six breast cancer cell lines was inhibited by both oils, with the *S. album* oil being more active than the *S. spicatum* oil by a factor of approximately 2-3.

### LUNG CANCER

Lung cancer cell lines were studied using the same experimental design described in Example 1. The results are presented below.

| Table 3. Activity against lung cancer cell lines | | | |
|---|---|---|---|
| Cell Line | Panel Name | IC₅₀ (µM) | |
| | | *Santalum album* | *Santalum spicatum* |
| A549 | Non-Small Cell Lung | 63 | 149 |
| EKVX | Non-Small Cell Lung | 48 | 186 |
| HOP-62 | Non-Small Cell Lung | 63 | 233 |
| HOP-92 | Non-Small Cell Lung | 126 | 193 |
| NCI-H226 | Non-Small Cell Lung | 62 | 108 |
| NCI-H23 | Non-Small Cell Lung | 56 | 99 |
| NCI-H322M | Non-Small Cell Lung | 66 | 114 |
| NCI-H460 | Non-Small Cell Lung | 45 | 98 |
| NCI-H522 | Non-Small Cell Lung | 49 | 85 |

These experiments showed robust activity against 9 cell lines derived from human non-small cell lung cancers. This indicates broad activity against many lung cancers. In particular, the growth of all nine lung cancer cell lines were inhibited by both oils, with the *S. album* oil being approximately 1.5-4 times more active than the *S. spicatum* oil.

### PROSTATE AND OVARIAN CANCER

Prostate and ovarian cancer cell lines were studied using the same experimental design described above. The results are presented below.

| Table 4. Activity against prostate and ovarian cancer cell lines | | | |
|---|---|---|---|
| Cell Line | Panel Name | IC₅₀ (µM) | |
| | | *Santalum album* | *Santalum spicatum* |
| OVCAR-3 | Ovarian | 18 | 54 |
| OVCAR-4 | Ovarian | 41 | 86 |
| OVCAR-5 | Ovarian | 64 | 117 |
| OVCAR-8 | Ovarian | 38 | 54 |
| NCI/ADR-RES | Ovarian | 59 | 105 |
| SK-OV-3 | Ovarian | 72 | 104 |
| PC-3 | Prostate | 35 | 90 |
| DU-145 | Prostate | 65 | 223 |

Robust activity against 8 cell lines derived from human ovarian and prostate cancers was shown. This indicates broad activity against many human gonadal cancers. In particular, the growth of all eight cancer cell lines were inhibited by both oils, with the *S. album* oil being approximately 1.5-3 times more active than the *S. spicatum* oil.

### RENAL CANCER

Renal cancer cell lines were studied using the experimental design described above. The results are presented below.

| Table 5 Activity against renal cancer cell lines | | | |
|---|---|---|---|
| Cell Line | Panel Name | IC₅₀ (µM) | |
| | | *Santalum album* | *Santalum spicatum* |
| 786-0 | Renal | 43 | 79 |
| A498 | Renal | 45 | 93 |
| ACHN | Renal | 78 | 279 |
| CAKI-1 | Renal | 68 | 212 |
| RXF 393 | Renal | 21 | 54 |
| SN12C | Renal | 38 | 63 |
| TK-10 | Renal | 46 | 90 |
| UO-31 | Renal | 60 | 106 |

Robust activity against 8 cell lines derived from human renal cancers was shown. This indicates broad activity against many livers cancers. In particular, the growth of all nine renal cancer cell lines were inhibited by both oils, with the *S. album* oil being approximately 2-4 times more active than the *S. spicatum* oil.

### OTHER CANCERS

Various other cancer cell lines were also studied using the experimental design described above. The results are presented below.

| Table 6 Activity against various cancer cell lines | | | |
|---|---|---|---|
| Cell Line | Panel Name | IC₅₀ (µM) | |
| | | *Santalum album* | *Santalum spicatum* |
| CCRF-CEM | Leukemia | 31 | 53 |
| HL-60(TB) | Leukemia | 6.6 | 7.8 |
| K-562 | Leukemia | 17 | 60 |
| MOLT-4 | Leukemia | 31 | 63 |
| RPMI-8226 | Leukemia | 16 | 33 |
| SR | Leukemia | 7 | 20 |
| COLO 205 | Colon | 51 | 251 |
| HCC-2998 | Colon | 69 | 118 |
| HCT-116 | Colon | 88 | 202 |
| HCT-15 | Colon | 49 | 78 |
| HT29 | Colon | 61 | 111 |
| KM12 | Colon | 64 | 101 |
| SW-620 | Colon | 14 | 44 |
| SF-268 | CNS | 23 | 61 |
| SF-295 | CNS | 31 | 129 |
| SF-539 | CNS | 22 | 76 |
| SNB-19 | CNS | 28 | 71 |
| SNB-75 | CNS | 39 | 122 |
| U251 | CNS | 26 | 78 |

Robust activity against various cell lines derived from human bone marrow, the CNS, and the colon was shown. This indicates broad activity against a large variety of cancers. In particular, the growth of all cancer cell lines were inhibited by both oils, with the *S. album* oil being more active than the *S*. *spicatum* oil in all lines (∼-1-3 fold in leukemia, ∼1.5-5 fold in colon, and ∼2.5-4 fold in CNS lines).

In summary, all cell lines studied had their growth inhibited by both oils with oil from *S. album* being more active than the oil from *S. spicatum.*

### DOSE RESPONSE CURVES

Several cells lines were examined over a 72 hour period to obtain dosages. The oils were evaluated against the SN**U**-398 hepatocellular carcinoma (HCC), FaDu Head and Neck cancer, HeLa cervical cancer, and the Mia**P**aCa-2 pancreatic cancer cell lines. Cell viability following 72-hour treatment was measured by MTT (3-(4,5-Dimethylthiazol-2-yl)-2,5-diphenyltetrazolium bromide) assay.

More particularly, cancer cell lines were obtained from American Type Culture Collection (ATCC) (Manassas, VA) and maintained in proper growth media. Cells were propagated at 37°C in a humidified atmosphere containing 5% carbon dioxide. Once grown to 50% confluency, cells were resuspended into cell culture media. One day prior to treatment (Day 0), cells are plated into wells of a 96-well plate for MTT assays.

Cell viability was determined using the MTT assay. This colorimetric procedure measures conversion of the MTT reagent to formazan by mitochondria. Formazan production was quantified by spectrophotometric measurement at 570 nm and is proportional to viable cell number. Cells were cultured and treated with different concentrations of the agent(s) for 72 hours. Following treatment, 50 µl of MTT was added to each well and allowed to incubate for 1-3 hours at 37°C. Each well was aspirated and 200 µl of DMSO added to each well to dissolve the formazan. Absorbance (OD) values were measured using a µQuant microplate reader at a single wavelength of 570 nm.

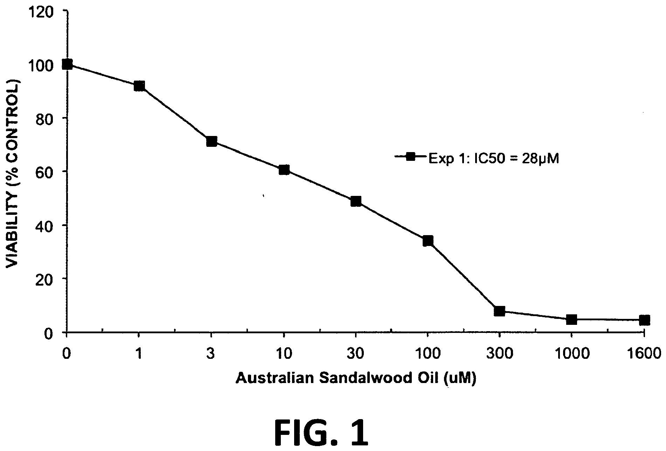

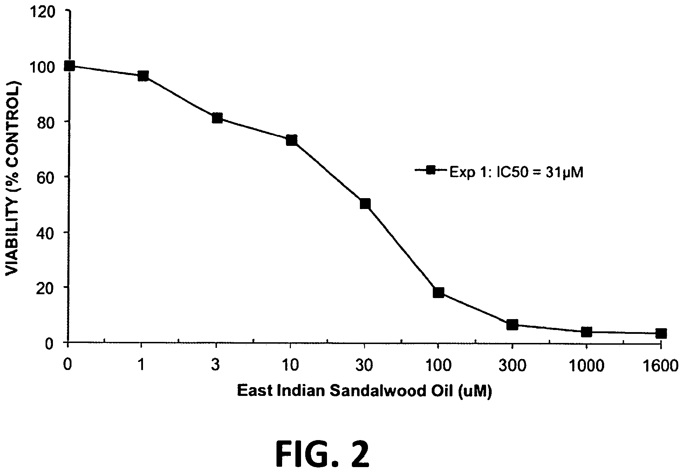

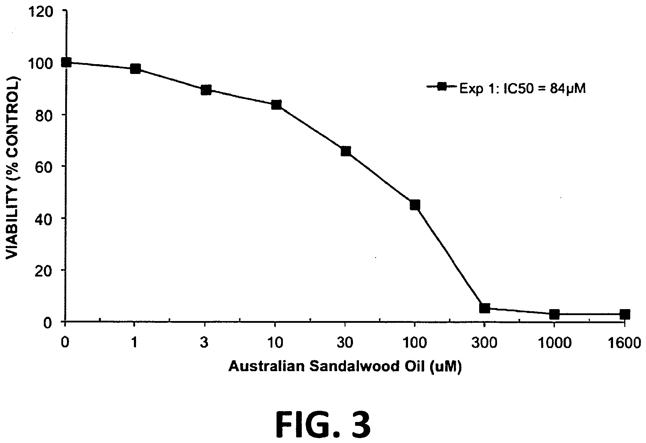

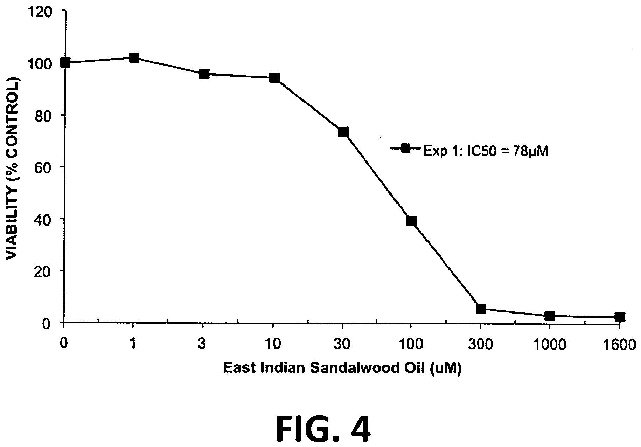

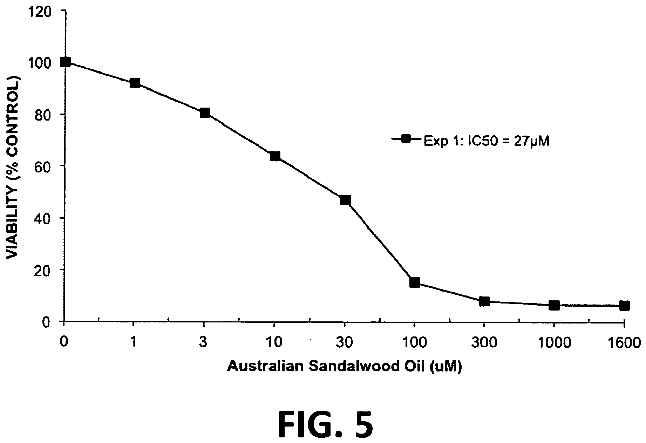

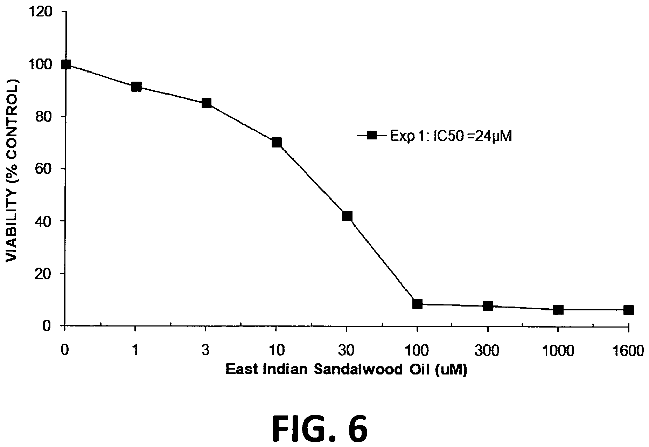

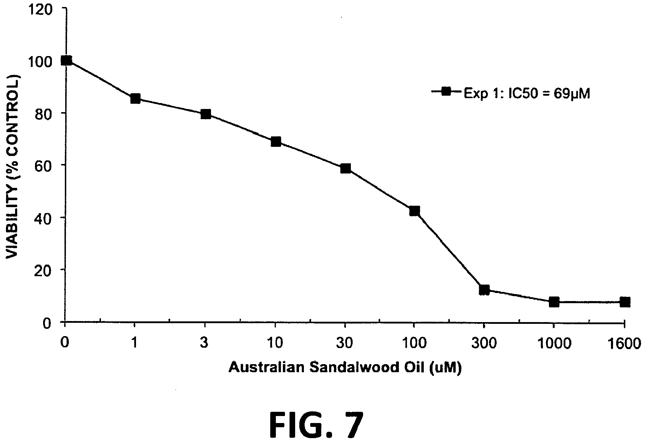

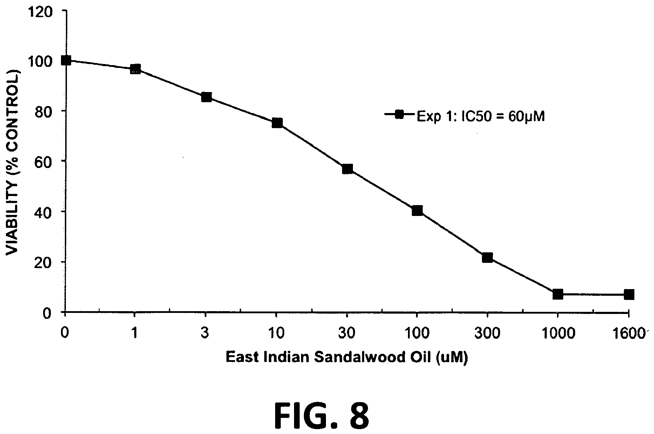

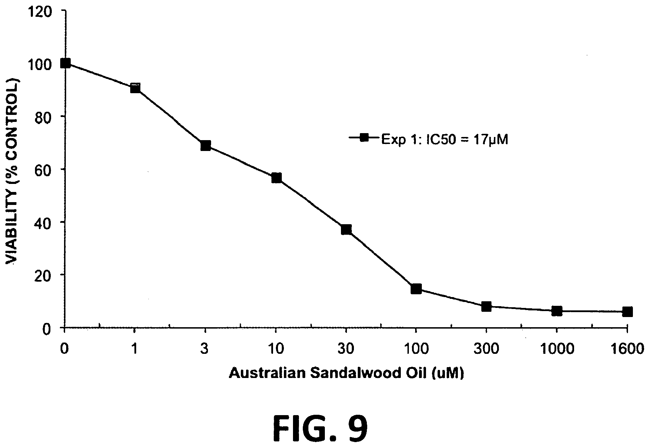

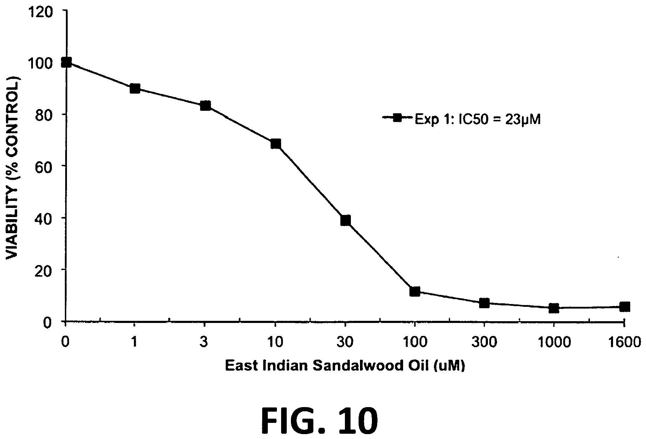

Cells were treated with sandalwood oil for 72 hours. Results from these studies were used to calculate an IC₅₀ value (concentration of drug that reduces cell viability by 50 percent of vehicle control for each agent) in each cell line. Statistical comparisons (t-test, (unpaired, two-tailed)) or ANOVA (between multiple groups) were made between each group. Differences are considered significant at p <0.05. The results are shown in Figures 1-10. Briefly, as was also seen in the NCI-60 Human Tumor Cell panel, the growth of all five cell lines were inhibited by both oils with the *S. album* oil being more active than the *S. spicatum* oil.

The results set forth above indicate that sandalwood oil has general applicability against cell lines that represent various solid tumors and other cancers. Therefore, as shown herein, sandalwood oil is a broad spectrum tumoricidal agent.

*In vivo* studies in mouse models utilize the oils formulated with simple, well characterized co-solvents (such as Cremophor) to show the ability of the oils to inhibit the growth of human tumor xenografts following systemic administration. Formulations based on liposomes, nanoparticles and microencapsulated oils can be evaluated *in vivo* as systemic delivery vehicles.

For example, and not to be limiting, the following protocol is used (See Jacob et al. Gene Ther. Mol. Biol. 8: 213-219 (2004)). Briefly, tumor cells are grown in complete medium. When cells are 70-80%, 3-4 hrs before harvesting, the medium is replaced with fresh medium to remove dead and detached cells. The medium is removed and cells are washed with PBS. A minimum amount of trypsin-EDTA is added. The cells are dispersed and complete medium is added (10:1 to 5:1). The cells are centrifuged immediately at or below 1500 rpm for 2-5 min and washed twice with PBS prior to storing the cells on ice. The cells are them counted using hemocytometer. Trypan blue staining is utilized to exclude dead cells. The cells are mixed 1:1 with trypan blue solution (Trypan Blue is dilute at 0.8 mM in PBS.) Viable cells exclude trypan blue, while dead cells stain blue due to trypan blue uptake. Cells are suspended in a volume so that 300 µl contains required number of cells per injection. Usually, about 3.0 x 10⁶ cells are needed per injection.

Mice that are 4-6 weeks old are injected subcutaneously (s.c.) into the lower flank with about 3.0 x 10⁶ cells. A composition comprising sandalwood is administered via any of the administration methods set forth herein after about 1-3 weeks when the tumors have reached an average volume of about 50-60 mm³. Tumor diameters are measured with digital calipers, and the tumor volume in mm³ is calculated by the formula: Volume = (width²) x length/2. After administration of the composition comprising sandalwood, the expected decrease in tumor size can be measured with digital calipers as described above. Decreases in symptoms can also be assessed.

### TOXICOLOGY

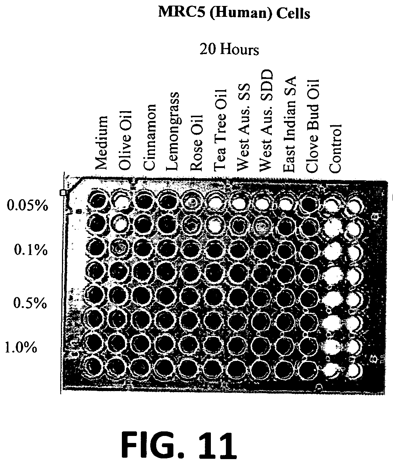

Figures 11 shows that sandalwood oil is not toxic in MRC5 cells at a concentration below about 0.1% sandalwood oil (w/w). In Figure 11, the light colored wells show no cell death and the darker wells show cell death in the human lung cells line MRC5 at 20 hours after administration of sandalwood oil. Figure 11 also shows that sandalwood oil is less toxic than other essential oils. For example, sandalwood oil is less toxic than cinnamon, lemongrass or clove bud oils in human MRC5 cells at a concentration less than about 0.1% (w/w). Sandalwood oil is also less toxic than cinnamon oil in human MRC5 cells at a concentration of about 0.05% (w/w), and between about 0.05% and about 0.1% (w/w). Sandalwood oil has a wider therapeutic window for treatment of cancer than most anti-tumorigenic agents. Therefore, sandalwood oil, even at high doses, higher than most other anti-tumorigenic agents, can be used to treat cancer with fewer or no deleterious effects to normal cells. Conversely, sandalwood oil, even at low doses, lower than most other anti-tumorigenic agents, has a therapeutic effect.

To obtain additional information regarding safety, mice can be dosed with increasing amounts of sandalwood oils to determine the LD₅₀ (See Opdyke et al., Food and Cosmetics Toxicology 989-990 (1974); and Bar and Griepentrog, Medizin Ernhar 244 (1967)). The Ames test can determine mutagenicity according to standard protocols (See McCann et al. "Detection of carcinogens as mutagens in the Salmonella/microsome test: Assay of 300 chemicals." Proc. Natl. Acad. Sci. USA 72(12): 5135-5139 (1975)).

### PREVENTING PROGRESSION OF ACTINIC KERATOSIS TO SQUAMOUS CELL CARCINOMA (for reference)

A topical formulation comprising sandalwood oil is administered to subject with an actinic keratosis lesion(s). The lesion is then observed visually and/or biopsied at time intervals, to determine if the lesion(s) has progressed to squamous cell carcinoma as compared to a control tissue sample. The tissue sample can be compared to a control tissue sample from an actinic keratosis lesion that was not contacted with the formulation or to a tissue sample from normal skin. Alternatively, the lesion can be biopsied and classified according to standard methods (**S**ee, for example, Krouse et al. "Progression of skin lesions from normal skin to squamous cell carcinoma," Anal. Quant. Cytol,. Histol. 31(1): 17-25 (2009)).

## Claims

1. Composition comprising sandalwood oil for use in treating a non-skin cancer in a subject, wherein the use comprises administration of an effective amount of said composition comprising sandalwood oil to the subject, wherein the sandalwood oil is sandalwood essential oil distilled or extracted from the wood of genus *Santalum* trees by steam distillation, hydrodistillation, or solvent extraction, wherein the subject has a non-skin cancer selected from the group consisting of a breast cancer, a head and neck cancer, an ovarian cancer, a bladder cancer, a prostate cancer, a lung cancer, a renal cancer, a leukemia, a liver cancer, a pancreatic cancer, a colon cancer, and a central nervous system cancer, and wherein the concentration of sandalwood oil in the composition is 0.0002-0.1% sandalwood oil (w/w).

2. The composition for use of claim 1, wherein the sandalwood oil is from *Santalum album* or *Santalum spicatum* or a combination thereof.

3. The composition for use of claim 1, wherein the sandalwood oil is from *Santalum album.*

4. The composition for use of claim 1, wherein the composition comprising sandalwood oil further comprises one or more pharmaceutically acceptable excipients.

5. The composition for use of claim 1, where the sandalwood oil comprises at least one of the components selected from the group consisting of E nerolidol, Alpha-santalene, Cis-alpha-(trans) bergamotene, Epi-beta-santalene, Beta-santalene, Gamma-curcumene, Dendrolasin, Alpha-santalol, Beta-bisabolol, Epi-alpha-bisabolol, Z-alpha trans-bergamotol, Epi beta-santalol, Cis-beta-santalol, E,E, farnesol, Cis nuciferol, Z-beta-curcumen-12-ol, and cis lanceol.

6. The composition for use of any of claims 1-5, wherein the composition comprises 1-300 micromolar sandalwood oil.

7. The composition for use of any of claims 1-6, wherein the composition comprises 0.0002-0.02% sandalwood oil (w/w).

8. The composition for use of any of claims 1-7, wherein the composition comprises a concentration of sandalwood oil that is less toxic than the same concentration of cinnamon oil in a cell, wherein the concentration of sandalwood oil is between 0.05% and 0.1%.

9. The composition for use of claim 8, wherein the cell is a human MRC5 cell.

10. The composition for use of claim 8, wherein the concentration of sandalwood oil is 0.0002-0.02% (w/w).

## Patentansprüche

1. Zusammensetzung, die Sandelholzöl aufweist, zur Verwendung bei der Behandlung eines Nicht-Hautkrebses in einem Wesen, wobei die Verwendung die Verabreichung einer wirksamen Menge der Zusammensetzung, die Sandelholzöl aufweist, an das Wesen umfasst, wobei das Sandelholzöl ätherisches Sandelholzöl ist, das aus Holz von Bäumen der Gattung *Santalum* durch Dampfdestillation, Hydrodestillation oder Lösungsmittelextraktion destilliert oder extrahiert wird, wobei das Wesen einen Nicht-Hautkrebs hat, ausgewählt aus der Gruppe bestehend aus Brustkrebs, Kopf-Hals-Krebs, Eierstockkrebs, Blasenkrebs, Prostatakrebs, Lungenkrebs, Nierenkrebs, Leukämie, Leberkrebs, Bauchspeicheldrüsenkrebs, Dickdarmkrebs, und Krebs des zentralen Nervensystems, und wobei die Konzentration von Sandelholzöl in der Zusammensetzung 0,0002-0,1% Sandelholzöl (w/w) ist.

2. Zusammensetzung zur Verwendung nach Anspruch 1, wobei das Sandelholzöl von *Santalum album* oder *Santalum spicatum* oder einer Kombination davon abstammt.

3. Zusammensetzung zur Verwendung nach Anspruch 1, wobei das Sandelholzöl von *Santalum album* abstammt.

4. Zusammensetzung zur Verwendung nach Anspruch 1, wobei die Zusammensetzung, die Sandelholzöl aufweist, ferner einen oder mehrere pharmazeutisch verträgliche Hilfsstoffe aufweist.

5. Zusammensetzung nach Anspruch 1, wobei das Sandelholzöl mindestens eine der Komponenten aufweist, die ausgewählt sind aus der Gruppe bestehend aus E-Nerolidol, alpha-Santalen, cis-alpha-(trans)Bergamoten, epi-beta-Santalen, beta-Santalen, gamma-Curcumen, Dendrolasin, alpha-Santalol, beta-Bisabolol, epi-alpha-Bisabolol, Z-alpha-trans-Bergamotol, epi-beta-Santalol, cis-beta-Santalol, E,E,Farnesol, cis-Nuciferol, Z-beta-Curcumen-12-ol, und cis-Lanceol.

6. Zusammensetzung zur Verwendung nach einem der Ansprüche 1 bis 5, wobei die Zusammensetzung 1-300 mikromolares Sandelholzöl aufweist.

7. Zusammensetzung zur Verwendung nach einem der Ansprüche 1 bis 6, wobei die Zusammensetzung 0,0002-0,02% Sandelholzöl (w/w) aufweist.

8. Zusammensetzung zur Verwendung nach einem der Ansprüche 1 bis 7, wobei die Zusammensetzung eine Konzentration von Sandelholzöl aufweist, die weniger toxisch ist als die gleiche Konzentration von Zimtöl in einer Zelle, wobei die Konzentration von Sandelholzöl zwischen 0,05% und 0,1 liegt %.

9. Zusammensetzung zur Verwendung nach Anspruch 8, wobei die Zelle eine menschliche MRC5-Zelle ist.

10. Zusammensetzung zur Verwendung nach Anspruch 8, wobei die Konzentration von Sandelholzöl 0,0002-0,02% (w/w) beträgt.

## Revendications

1. Composition comprenant de l'huile de bois de santal destinée à être utilisée dans le traitement d'un cancer autre que le cancer de la peau chez un sujet, où l'utilisation comprend une administration au sujet d'une quantité efficace de ladite composition comprenant de l'huile de bois de santal, où l'huile de bois de santal est une huile essentielle de bois de santal distillée ou extraite à partir du bois d'arbres du genre *Santalum* par distillation par entraînement à la vapeur, par hydrodistillation, ou par extraction au solvant, où le sujet est atteint d'un cancer autre que le cancer de la peau sélectionné parmi le groupe constitué d'un cancer du sein, d'un cancer de la tête et du cou, d'un cancer de l'ovaire, d'un cancer de la vessie, d'un cancer de la prostate, d'un cancer des poumons, d'un cancer du rein, d'une leucémie, d'un cancer du foie, d'un cancer du pancréas, d'un cancer du côlon, et d'un cancer du système nerveux central, et où la concentration d'huile de bois de santal dans la composition est comprise entre 0,000 2 et 0,1 % (m/m) d'huile de bois de santal.

2. Composition destinée à être utilisée selon la revendication 1, dans laquelle l'huile de bois de santal provient de *Santalum album* ou de *Santalum spicatum* ou d'une combinaison de ceux-ci.

3. Composition destinée à être utilisée selon la revendication 1, dans laquelle l'huile de bois de santal provient de *Santalum album.*

4. Composition destinée à être utilisée selon la revendication 1, où la composition comprenant de l'huile de bois de santal comprend en outre un ou plusieurs excipients pharmaceutiquement acceptables.

5. Composition destinée à être utilisée selon la revendication 1, dans laquelle l'huile de bois de santal comprend au moins un des composants sélectionnés parmi le groupe constitué de l'E-nérolidol, de l'alpha-santalène, du cis-alpha-(trans)-bergamotène, de l'épi-bêta-santalène, du bêta-santalène, du gamma-curcumène, de la dendrolasine, de l'alpha-santalol, du bêta-bisabolol, de l'épi-alpha-bisabolol, du Z-alpha-trans-bergamotol, de l'épi-bêta-santalol, du cis-bêta-santalol, de l'E,E-farnesol, du cis-nuciférol, du Z-bêta-curcumène-12-ol, et du cis-lancéol.

6. Composition destinée à être utilisée selon l'une quelconque des revendications 1 à 5, où la composition comprend entre 1 et 300 micromolaire d'huile de bois de santal.

7. Composition destinée à être utilisée selon l'une quelconque des revendications 1 à 6, où la composition comprend entre 0,000 2 et 0,02 % (m/m) d'huile de bois de santal.

8. Composition destinée à être utilisée selon l'une quelconque des revendications 1 à 7, où la composition comprend une concentration d'huile de bois de santal qui est moins toxique que la même concentration d'huile de cinnamome dans une cellule, où la concentration d'huile de bois de santal est comprise entre 0,05 % et 0,1 %.

9. Composition destinée à être utilisée selon la revendication 8, où la cellule est une cellule MRC5 humaine.

10. Composition destinée à être utilisée selon la revendication 8, dans laquelle la concentration d'huile de bois de santal est comprise entre 0,000 2 et 0,02 % (m/m).
